# EUROPÄISCHE PATENTANMELDUNG

(11) **EP 1 612 709 A2**
(43) Veröffentlichungstag der Anmeldung: **04.01.2006**
(21) Anmeldenummer: 05105641.4
(22) Anmeldetag: 23.06.2005
(51) Int. Cl.: G06F 19/00

(54) **Zeitmanagementsystem für medizinische Anwendungen**

(30) Priorität: 30.06.2004 DE 102004031690; 12.04.2005 DE 102005016852
(71) Anmelder: SIEMENS AKTIENGESELLSCHAFT, 80333 München (DE)
(72) Erfinder: Wendl, Udo, 91334 Hemhofen (DE); Wyczisk, Heintje, 91054 Erlangen (DE)

(57) **Zusammenfassung**

Zur Rationalisierung medizinischer Behandlungen im klinischen Umfeld, insbesondere zur Verbesserung der Auslastung der Untersuchungs- und Therapiegeräte und zur Vermeidung langer Wartezeiten, wird ein Zeitmanagementsystem (1) mit mindestens einer Behandlungsstation (2a-2c) sowie mit einem Terminplanungsmodul (3) zur Speicherung von Terminen (T1,T2), deren jeder einem in Hinblick auf die oder eine bestimmte Behandlungsstation (2a-2c) und eine Vorgabedauer (tv) spezifizierten medizinischen Ablauf (8a-8c) zugeordnet ist, angegeben. Die Behandlungsstation (2a-2c) ist dazu ausgebildet, bei vollendeter Durchführung eines Ablaufs (8a-8c) ein Rückmeldungssignal (R) an das Terminplanungsmodul (3) auszugeben. Das Terminplanungsmodul (3) ist dazu ausgebildet, nachfolgende Termine (T2) anhand des Rückmeldungssignals (R) dynamisch anzupassen.

## Beschreibung

Die Erfindung bezieht sich auf ein Zeitmanagementsystem für medizinische Behandlungen, das insbesondere zum Einsatz in einer Klinik vorgesehen ist.

Im klinischen Umfeld werden medizinische Behandlungen, ähnlich zu industriellen Prozessen, zunehmend in Form von standardisierten Abläufen (oder Workflows) definiert. Der Begriff Behandlung umfasst hierbei das gesamte Umfeld medizinischer Handlungen, insbesondere zur Diagnose, Therapieplanung und Therapie. Die Effizienz einer solchen Standardisierung gewinnt insbesondere unter dem zunehmenden Kostendruck und der hierdurch in steigenden Maße erforderlichen Wirtschaftlichkeit einer Klinik zunehmend an Bedeutung. Sind standardisierte Abläufe vordefiniert, können diese besser geplant und mit Terminen versehen werden. Anders als bei gewöhnlichen industriellen Prozessen gestaltet sich die Ausführung eines solchen Terminplanes im klinischen Umfeld jedoch in der Regel schwierig. Dies ist insbesondere darauf zurückzuführen, dass die tatsächliche Dauer einer medizinischen Behandlung aufgrund der individuellen Verschiedenheit eines jeden untersuchten Patienten und des zugehörigen Krankheitsbildes vorab nur mit vergleichsweise großer Unsicherheit abzuschätzen ist. Insbesondere werden Erkenntnisse, die die Dauer der Behandlung beeinflussen, regelmäßig erst im Laufe einer Behandlung bekannt.

Die hierdurch verursachten Abweichungen gegenüber dem ursprünglichen Terminplan führen, je nach dem Vorzeichen der Abweichung zu langen Wartezeiten für nachfolgende Patienten oder zu Standzeiten der Untersuchungsgeräte zwischen zwei Behandlungen und damit zu einem vergleichsweise geringem Auslastungsgrad. Eine geringe Auslastung stellt vor allem bei einer kostenintensiven Untersuchungs- oder Behandlungseinrichtung, insbesondere einem zu Therapiezwecken eingesetzten Teilchenbeschleuniger, einen erheblichen Nachteil dar.

Zur Terminplanung im klinischen Umfeld wird bisher üblicherweise ein gebräuchlicher, insbesondere software-implementierter Terminplaner mit statischer Zeiteinteilung verwendet. Ein solcher Terminplaner erlaubt die Vergabe von Terminen innerhalb fester oder variabler Zeitfenster. Kommt es in der Praxis zu Abweichungen von dem Terminplan, so muss der Terminplan gegebenenfalls in aufwendiger Weise manuell aktualisiert werden. Unterbleibt, z. B. aus Zeitgründen, die manuelle Aktualisierung, führt dies dazu, dass sich eine einmalige Verzögerung auch auf neu vergebene Termine immer weiter fortpflanzt.

Der Erfindung liegt die Aufgabe zugrunde, ein Zeitmanagementsystem für medizinische Behandlungen im klinischen Umfeld anzugeben, welches den vorstehend beschriebenen Problemen entgegenwirkt.

Diese Aufgabe wird erfindungsgemäß gelöst durch die Merkmale des Anspruchs 1. Danach umfasst das Zeitmanagementsystem mindestens eine Behandlungsstation sowie ein Terminplanungsmodul. Bei der Behandlungsstation handelt es sich vorrangig um eine einem Teilchenbeschleuniger (Linearbeschleuniger, Zyklotron oder Synchrotron) zugeordnete medizinische Bestrahlungseinrichtung oder eine medizinische Untersuchungsvorrichtung, z.B. einen Computertomographen, eine sonstige Röntgenvorrichtung, einen Magnetresonanztomographen o. dgl. Als Behandlungsstation kann ferner jedoch auch ein einem Behandlungszimmer oder Operationssaal zugeordneter Rechner vorgesehen sein. Das Terminplanungsmodul ist bevorzugt als Softwarebestandteil einer innerhalb eines Datennetzes mit jeder Behandlungsstation verbundenen Datenverarbeitungsanlage ausgeführt. Das Terminplanungsmodul ist hierbei dazu ausgebildet, eine Anzahl von Terminen zu speichern, deren jeder einem medizinischen Ablauf zugeordnet ist. Als Ablauf wird ein Schema einer medizinischen Behandlung bezeichnet, das insbesondere hinsichtlich einer zugehörigen Behandlungsstation und einer Vorgabedauer, d.h. einer durchschnittlichen Soll-Dauer spezifiziert ist. Durch Terminierung eines solchen Ablaufes, d.h. durch Erzeugung eines einem bestimmten Ablaufs zugeordneten Termins, wird dem Ablauf eine Anfangszeit sowie ein zu untersuchender Patient zugeordnet. Durch den Termin und den zugeordneten Ablauf sind somit alle für eine medizinische Behandlung wesentlichen Daten festgelegt, insbesondere Anfangszeit, Vorgabedauer, Patient, Art der Behandlung und Behandlungsstation.

Die oder jede Behandlungsstation ist nun erfindungsgemäß dazu ausgebildet, bei Vollendung eines termingemäß durchgeführten Ablaufs ein Rückmeldungssignal an das Terminplanungsmodul auszugeben, wobei das Terminplanungsmodul wiederum dazu ausgebildet ist, die Anfangszeiten nachfolgender Termine anhand des Rückmeldungssignals dynamisch anzupassen. Dies umfasst zunächst eine Verschiebung von Terminen. Überschreitet die termingemäße Durchführung eines Ablaufs die dem Ablauf zugeordnete Vorgabezeit, so werden infolge dieser dynamischen Anpassung nachfolgende Termine entsprechend verzögert. Unterschreitet die termingemäße Durchführung des Ablaufs dagegen die Vorgabezeit, so werden nachfolgende Termin, die der Behandlungsstation zugeordnet sind, entsprechend vorgezogen. Gegebenenfalls kann auch die Reihenfolge von Terminen oder, bei mehreren äquivalenten Behandlungsstationen, die einem Termin zugeordnete Behandlungsstation nach Bedarf angepasst werden. Ferner ist vorgesehen, auch die Zuteilung von Ressourcen (z.B. den Teilchenstrahl eines Teilchenbeschleunigers) und die Personalplanung für die Behandlungsstationen entsprechend dem Terminplan dynamisch anzupassen.

Durch die Rückkopplung der oder jeder Behandlungsstation mit dem Terminplanungsmodul und die durch Letzteres vorgenommene dynamische Anpassung des Terminplans ist gewährleistet, dass der Terminplan fortlaufend automatisch aktualisiert wird. Hierdurch wird einerseits eine besonders gute Auslastung der Behandlungsstationen erreicht, andererseits können auf diese Weise lange Wartezeiten wirksam vermieden werden. Insbesondere kann auf einfache Weise sichergestellt werden, dass die Neuvergabe von Terminen stets im Hinblick auf eine aktualisierte Fassung des bestehenden Terminplans vorgenommen wird. Hierdurch wird wirksam vermieden, dass eine einmalige Verzögerung sich kettenreaktionsartig auf immer neue Termine fortpflanzt.

Im Hinblick auf eine effiziente und rationelle Terminvergabe ist bevorzugt vorgesehen, dass dem Terminplanungsmodul zu der oder jeder Behandlungsstation mindestens ein vordefinierter Ablauf vorgegeben ist, der bereits von vornherein hinsichtlich der ihm zugeordneten Vorgabedauer spezifiziert ist. Alternativ kann jedoch auch zumindestens für einige Abläufe vorgesehen sein, dass die Vorgabedauer erst bei der Terminierung manuell festgelegt wird.

In einer Verfeinerung des Zeitmanagementsystems ist vorgesehen, dass der oder jeder Ablauf in eine Anzahl von Ablaufschritten unterteilt ist. In dieser Ausführung ist zweckmäßigerweise jede Behandlungsstation dazu ausgebildet, die Vollendung eines jeden termingemäß durchgeführten Ablaufschritts durch Ausgabe eines entsprechenden Rückmeldungssignals an das Terminplanungsmodul anzuzeigen, so dass dieses gegebenenfalls schon während einer laufenden Behandlung auf etwaige Abweichungen gegenüber dem ursprünglichen Terminplan reagieren und den Terminplan entsprechend anpassen kann.

In einer besonders vorteilhaften Ausführung der Erfindung ist das Zeitmanagementsystem auf Grundlage des DICOM-Standards ausgeführt. Der in der bildgebenden Medizintechnik international etablierte DICOM(Digital Imaging and Communications in Medicine)-Standard definiert Datenobjekte, Kommunikationsprotokolle und Server/Client-Anwendungen im diagnostischen Umfeld. So werden darin z.B. Datenobjekte definiert, die ein Röntgenbild, Computertomogramm oder MR-Bild darstellen. Der Standard definiert weiterhin, wie diese Objekte z.B. von dem Aufnahmegerät (Erzeuger) an ein Auswertegerät (Workstation) oder ein digitales Archiv übermittelt werden. Im Hinblick auf weitere Details wird auf die Dokumentation des Standards "Digital Imaging and Communications in Medicine (DICOM)", Version 3, Part 1 (PS 3.1-2003) bis Part 16 (P S 3.16-2003), NEMA, Rosslyn, Virginia (USA) Bezug genommen. Die Server/Client-Definition gemäß DICOM wird ergänzt durch den IHE(Integrating the Healthcare Enterprise)-Standard, der die Rollen und Objekte vorsieht, mit denen die an klinischen Prozessen beteiligten Komponenten, z.B. Radiologieinformationssystem (RIS), Aufnahmegerät und Auswertestation, ihre Aufgaben im Prozess zugewiesen bekommen. So kann beispielsweise das RIS eine Liste von zu untersuchenden Patienten generieren. Diese Liste holt sich das Aufnahmegerät zusammen mit den relevanten Patientendaten ab. Nach erfolgter Aufnahme der Untersuchungsdaten, z.B. eines Röntgenbildes, wird die komplette Information an eine Befundungsstation in der Radiologie weitergeleitet. Der Radiologe diktiert dann den Befund. Die Bilder werden in einem digitalen Archiv gespeichert. Das RIS speichert die Befunde.

Der DICOM-Standard bietet nun neben bereits vorgegebenen und international standardisierten Abläufen auch die Möglichkeit, frei konfigurierbare Abläufe zu definieren. Diese Server/Client-Anwendungen sind unter dem Begriff "General Purpose Worklist oder General Purpose Procedure Steps (GPPS)" zusammengefasst. Mit GPPS lassen sich also beliebige, mit dem DICOM-Standard kompatible Prozesse im klinischen Umfeld beschreiben. DICOM stellt hierzu insbesondere die Klasse der Planned-Procedure-Steps zur Verfügung, mittels derer auszuführende Prozessschritte definiert werden können. DICOM stellt weiterhin die Klasse der Performed-Procedure-Steps zur Verfügung, mittels welcher die an dem klinischen Prozess beteiligten Komponenten die Ausführung der Planned-Procedure-Steps an einen Procedure-Step-Provider zurückmelden können. Mit Hilfe dieser, durch den DICOM-Standard für alle Hersteller verfügbaren Objekt- und Kommunikationsdefinitionen können nun DICOM-kompatible Komponenten Rückmeldungen liefern. Erkanntermaßen lässt sich die GPPS-Umgebung besonders vorteilhaft zur Realisierung des dynamisch adaptiven Zeitmanagement anwenden. Hierzu ist vorgesehen, dass das Terminplanungsmodul zur Terminierung eines Ablaufs den oder jeden Ablaufschritt des Ablaufs in Form eines Planned-Procedure-Steps gemäß DICOM formuliert und der dem Ablauf zugehörigen Behandlungsstation zuweist. Zur Realisierung des Rückmeldungssignals ist zweckmäßigerweise vorgesehen, dass die oder jede Behandlungsstation jeden termingemäß durchgeführten Ablaufschritt in Form eines Performed-Procedure-Steps gemäß DICOM formuliert und an das Terminplanungsmodul rückmeldet. Durch Überwachung dieser rückgemeldeten Performed-Procedure-Steps ist nun eine einfache Anpassung des Terminplans möglich.

Zusätzlich oder alternativ hierzu ist vorgesehen, zur Generierung von Rückmeldungssignalen ein zur Lokalisation von Patienten, Transportgeräten oder medizinischen Verbrauchsgütern ausgebildetes Lokalisierungssystem heranzuziehen. Das Lokalisationssystem kann auf Basis eines Strichcodesystems realisiert sein. Bevorzugt wird aber zur Generierung von Rückmeldungssignalen RFID-(Radiofrequenzidentifikations-)oder auch Transponder-Technik eingesetzt. Hierbei umfasst das Lokalisationssystem mindestens einen RFID-Signalgeber und mindestens ein RFID-Lesegerät, das das Rückmeldungssignal generiert, wenn der bzw. ein RFID-Signalgeber von dem Lesegerät ausgelesen wird. Der RFID-Signalgeber ist insbesondere als mobiles Teil ausgebildet und einem Transportgerät (insbesondere einem Transportwagen), einem medizinischen Verbrauchsgut (als Teil einer Packung, als Aufkleber auf einer solchen, o.dgl.) oder direkt einem Patienten (z.B. als Identifikationsplakette) zugeordnet. RFID-Signalgeber oder Transponder können am Patienten (Arm, Bein), Behandlungshilfen (z.B. Vakuummatratzen) oder auch am Behandlungstisch (Rollwagen/Liege, Rollstuhl) fixiert werden. Die Lesegeräte sind entsprechend an- und auszurichten. Das oder jedes Lesegerät ist dagegen bevorzugt stationär, d.h. einem bestimmten Ort zugeordnet, so dass die aktuelle Position des Patienten, Transportgeräts bzw. Verbrauchsguts innerhalb einer Klinik lokalisiert wird, wenn der zugehörige RFID-Signalgeber in den Einflussbereich eines an diesem Ort stationierten Lesegeräts gebracht wird. Die Lesegeräte sind insbesondere auf verschiedener Höhe über dem Fußboden angebracht, so dass sowohl an Armen und Beinen stehender oder gehender Patienten als auch an liegenden Patienten befestigte Signalgeber bzw. Transponder eingelesen werden können. Beispielsweise wird bei Betreten oder Verlassen einer Behandlungsstation, einer Umkleidekabine, etc. durch Kommunikation des einem Patienten zugeordneten RFID-Signalgebers mit einem entsprechenden Lesegerät automatisch ein Rückmeldungssignal erzeugt.

Das aus der Zuordnung von Ort und Zeit generierte Rückmeldesignal wird mittels vordefinierten medizinischen Abläufen (Workflow, Procedure Step) dem Behandlungsschritt zugewiesen und darauf basierend eine Optimierung der Terminierung durch das Terminplanungsmodul vorgenommen.

Die Terminierung eines Ablaufs erfolgt optional manuell, indem das medizinische Personal einen vordefinierten Ablauf auswählt, den zu untersuchenden Patienten spezifiziert und insbesondere eine dem Termin zugeordnete Anfangszeit auswählt. Bevorzugt ist das Terminplanungsmodul jedoch dazu ausgebildet, die dem Termin zugeordnete Zeit automatisch auszuwählen und, gegebenenfalls nach positiver Quittierung durch das medizinische Personal, in den Terminplan einzutragen. Das Terminplanungsmodul ist dabei derart ausgebildet, dass es die dem Termin zugeordnete Anfangszeit nach Maßgabe einer optimierten Auslastung der zugehörigen Behandlungsstation auswählt. Sind mehrere Behandlungsstationen vorhanden, die von gemeinsamen Ressourcen abhängig sind, so ist zweckmäßigerweise vorgesehen, dass das Terminplanungsmodul die Termine der verschiedenen Behandlungsstationen derart zeitlich zueinander anordnet, dass die gemeinsam genutzten Ressourcen gleichmäßig ausgelastet sind. Kommt es zu einer Verzögerung im Bereich einer Behandlungsstation, so passt das Terminplanungsmodul zweckmäßigerweise auch die nachfolgenden Termine der anderen Behandlungsstationen entsprechend an. Optional wird die Terminierung unter Berücksichtigung einer statistischen Vorkorrektur vorgenommen, die aus einer Auswertung bereits erfolgter Terminänderungen abgeleitet wird. Tritt beispielsweise bei einem bestimmten Ablauf statistisch mit außergewöhnlicher Häufigkeit eine Verzögerung auf, so wird das Terminplanungsmodul bei der zukünftigen Terminierung dieses Ablaufs eine längere Zeitspanne als die dem Ablauf ursprünglich zugeordnete Vorgabedauer einplanen.

Für eine verbesserte Wartezeitenvermeidung ist zweckmäßigerweise vorgesehen, dass das Terminplanungsmodul bei einer dynamischen Anpassung eines Termins den zugeordneten Patienten nach vordefinierten Kriterien automatisch benachrichtigt. Dies kann in Form einer direkten Benachrichtigung erfolgen, z.B. durch einen automatisch generierten Anruf, eine SMS, mittels eines Funkrufempfängers ("Pieper"), etc. Alternativ kann der Patient aber auch mittelbar, z.B. durch Benachrichtigung des für den Patienten verantwortlichen Betreuungspersonals, benachrichtigt werden. Des Weiteren ist denkbar, dass die Termine den Patienten in stets aktualisierter Form auf einer Anzeigetafel, einem Informationsbildschirm o. dgl. zugänglich gemacht werden.

Zusätzlich oder alternativ hierzu ist vorgesehen, dass der einem Termin zugeordnete Patient durch das Terminplanungsmodul automatisch um eine vorgegebene Vorlaufspanne vor der dem Termin zugewiesenen Zeit automatisch benachrichtigt wird. Diese Option ist insbesondere im stationären klinischen Umfeld von großem Vorteil, zumal hier ein Patient innerhalb einer vergleichsweise kurzen Zeitspanne zu einer Behandlung abrufbar ist, so dass Wartezeiten fast gänzlich vermieden werden können.

Nachfolgend wird ein Ausführungsbeispiel der Erfindung anhand einer Zeichnung näher erläutert. Darin zeigen:
- FIG 1: in einem schematisch vereinfachten Blockschaltbild ein Zeitmanagementsystem mit einem Terminplanungsmodul, drei Behandlungsstationen und einer Anzahl von Kommunikationseinheiten zur Kommunikation mit einem Patienten,
- FIG 2: in einem vereinfachten Funktionsschema das Terminplanungsmodul bei der Terminierung eines medizinischen Ablaufs,
- FIG 3: in einem weiteren Funktionsschema das Terminplanungsmodul bei einer dynamischen Terminanpassung, und
- FIG 4: in Darstellung gemäß FIG 1 eine Variante des Zeitmanagementsystems mit einem Lokalisierungssystem auf RFID-Basis.

Einander entsprechende Teile und Größen sind in allen Figuren stets mit den gleichen Bezugszeichen versehen.

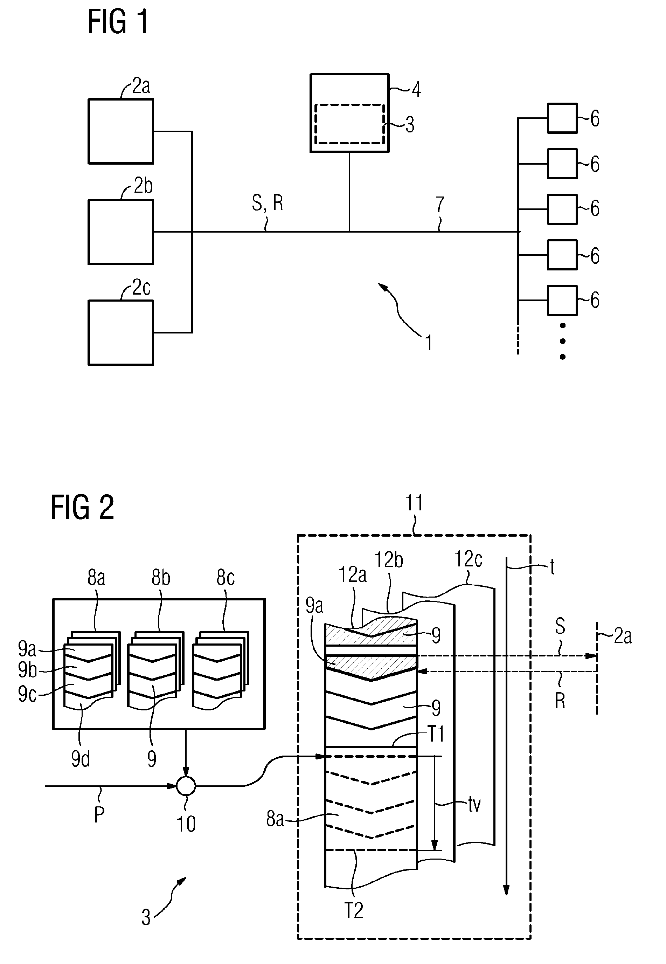

In FIG 1 ist schematisch vereinfacht ein Zeitmanagementsystem 1 dargestellt, das insbesondere zum Einsatz in einer Klinik vorgesehen ist. Das Zeitmanagementsystem 1 umfasst drei Behandlungsstationen 2a,2b,2c. Bei den Behandlungsstationen 2a-2c handelt es sich um medizinische Therapie- und Untersuchungsvorrichtungen, beispielsweise eine Röntgenvorrichtung, eine Bestrahlungsstation eines Teilchenbeschleunigers und einen Magnetresonanztomographen. Ferner kann auch ein einem Untersuchungsraum oder Operationsraum zugeordneter Computer eine Behandlungsstation bilden.

Das Zeitmanagementsystem 1 umfasst weiterhin ein Terminplanungsmodul 3, das als Softwarebestandteil einer Datenverarbeitungsanlage 4, z.B. eines zentralen Servers, ausgeführt ist.

Das Zeitmanagementsystem 1 nimmt außerdem Zugriff auf eine Anzahl von Kommunikationseinheiten 6 zur Kommunikation mit den Patienten. Als Kommunikationseinheit 6 kann ein Kommunikationsmittel herangezogen werden, mittels welchem ein Patient oder das medizinische Betreuungspersonal direkt ansprechbar ist, z. B. ein Zimmer- oder Stationstelephon, Mobiltelephon, etc. Als Kommunikationseinheit 6 kann aber auch ein allgemein zugängliches Informationsmedium, z. B. eine Anzeigetafel, ein Informationsbildschirm oder dergleichen einbezogen sein.

Die Behandlungsstationen 2a-2c, die Datenverarbeitungsanlage 4 und die Kommunikationseinheiten 6 sind untereinander über ein Datenübertragungsnetzwerk 7 verbunden. Das Datenübertragungsnetzwerk 7 kann auch mehrere getrennte Netze umfassen, z.B. ein LAN, ein Tk-Netz sowie gegebenenfalls Funkstrecken etc.

Aufgabe des in FIG 2 in einem Funktionsschema dargestellten Terminplanungsmoduls 3 ist es, Behandlungstermine der Patienten an den Behandlungsstationen 2a-2c derart zu koordinieren, dass die Behandlungsstation 2a-2c einerseits möglichst gut ausgelastet sind, d.h. insbesondere Standzeiten während der gewöhnlichen Behandlungszeiten weitgehend vermieden sind, wobei andererseits lange Wartezeiten für die Patienten vermieden sein sollen.

Dem Terminplanungsmodul 3 sind hierzu zu jeder Behandlungsstation 2a-2c eine Anzahl von vordefinierten Abläufen 8a,8b bzw. 8c vorgegeben. Jeder Ablauf 8a-8c stellt das abstrakte Schema einer medizinischen Behandlung dar, welche an der zugehörigen Behandlungsstation 2a,2b bzw. 2c zur Durchführung vorgesehen ist. Handelt es sich bei der Behandlungsstation 2a beispielsweise um eine Röntgenvorrichtung, so ist unter den dieser Behandlungsstation 2a zugeordneten Abläufen 8a beispielsweise ein Ablauf 8a auf eine Thorax-Untersuchung in Aufnahmeprojektion anterior-posterior gerichtet. Handelt es sich bei der Behandlungsstation 2b beispielsweise um eine Bestrahlungsstation, so ist ein zugeordneter Ablauf 8b beispielsweise auf eine bestimmte Bestrahlung gerichtet.

Jeder Ablauf 8a-8c ist wiederum gegliedert in eine Anzahl von Ablaufschritten 9. Die sukzessive aufeinanderfolgenden Ablaufschritte 9a-9d des Ablaufs 8a beinhalten beispielsweise die Registrierung eines Patienten (9a), die Immobilisierung des Patienten in der Aufnahmeposition (9b), die eigentliche Bildaufnahme (9c) und die anschließende Remobilisierung des Patienten (9d). Jedem Ablaufschritt 9,9a-9d ist hierbei eine vorgegebene Vorgabezeit, d.h. eine durchschnittliche Sollzeit, zugewiesen. Aus der Summe der den einzelnen Ablaufschritten 9,9a-9d eines Ablaufs 8a-8c zugewiesenen Vorgabezeiten ergibt sich insbesondere eine dem entsprechenden Ablauf zugewiesene (Gesamt-)Vorgabedauer tv, die in FIG 2 schematisch angedeutet ist.

Im Zuge der Terminplanung werden Abläufe 8a-8c durch das Terminplanungsmodul 3 terminiert, d.h. einem bestimmten Ablauf 8a-8c wird ein Termin T1,T2 zugewiesen. Ein Termin T1,T2 stellt eine konkrete Instanz eines abstrakten Ablaufs 8a-8c dar, durch welche der Ablauf 8a-8c hinsichtlich eines bestimmten Patienten und einer zugewiesenen Anfangszeit ta konkretisiert wird. Der Termin T1,T2 spezifiziert somit alle für die konkrete Ausführung einer dem entsprechenden Ablauf 8a-8c entsprechenden medizinischen Behandlung erforderlichen Informationen.

Zur Terminierung eines Ablaufes 8a-8c wählt das medizinische Personal den entsprechenden Ablauf 8a-8c aus und spezifiziert den zu untersuchenden Patienten durch Eingabe entsprechender Patientendaten P, die eine Identifizierung des Patienten erlauben, z.B. den Namen des Patienten oder eine dem Patienten entsprechende Kennnummer etc. Die dem Termin T1,T2 zugewiesene Anfangszeit ta wird entweder ebenfalls manuell durch das medizinische Personal spezifiziert oder automatisch durch ein Terminvergabemodul 10 des Terminplanungsmoduls 3 festgelegt. Der Termin T1,T2 wird anschließend durch das Terminvergabemodul 10 in einen Terminplan 11 des Terminplanungsmoduls 3 eingetragen. Der Terminplan 11 ist ein Kalender mit einer Anzahl von Feldern 12a-12c, deren jedes einer Behandlungsstation 2a,2b bzw. 2c zugeordnet ist. Die Zeitachse t des Kalenders ist in FIG 2 schematisch als Pfeil angedeutet.

FIG 2 zeigt schematisch die Festsetzung des Termins T2, der einem der Behandlungsstation 2a zugeordneten Ablauf 8a zugewiesen ist. Der Termin T2 wird entsprechend in das Feld 12a des Terminplans 11 derart eingetragen, dass er zeitlich unmittelbar an den letzten bereits bestehenden Termin T1 angrenzt.

Optional ist vorgesehen, dass das Terminvergabemodul 10 nach erfolgter Terminvergabe den entsprechenden Patienten automatisch benachrichtigt.

Zusätzlich zu der Vergabe von Terminen T1, T2 steuert und überwacht das Terminplanungsmodul 3 die konkrete Durchführung der entsprechenden Abläufe 8a-8c durch die zugehörigen Behandlungsstationen 2a-2c. Hierzu gibt das Terminplanungsmodul 3 zu jedem termingemäß durchzuführenden Ablaufschritt 9 ein entsprechendes Steuersignal S an die zugehörige Behandlungsstation 2a-2c ab. Nach erfolgter Durchführung dieses Ablaufschritts 9 gibt die Behandlungsstation 2a-2c ein entsprechendes Rückmeldungssignal R an das Terminplanungsmodul 3 zurück. Das Terminplanungsmodul 3 erkennt durch Auswertung der Rückmeldungssignale R bereits abgearbeitete Ablaufschritte 9,9a (in FIG 2 schraffiert angedeutet).

In bevorzugter Ausführung nutzt das Zeitmanagementsystem 1 zur Bildung der Steuersignale S und Rückmeldungssignale R die durch den DICOM-Standard definierten Klassen der sogenannten General Purpose Procedure Steps (GPPS). Das Terminplanungsmodul erzeugt danach Steuersignale S, indem es einen auszuführenden Ablaufschritt (in FIG 2 exemplarisch hervorgehoben der Ablaufschritt 9a des Termins T1) als Planned-Procedure-Step formuliert und der Behandlungsstation 2a zugänglich macht. Die Behandlungsstation 2a erzeugt gemäß FIG 2 das Rückmeldungssignal R, indem sie nach termingerechter Durchführung des Ablaufschritts 9a diesen als Performed-Procedure-Step formuliert und das Terminplanungsmodul 3 zurückgibt. In das Zeitmanagementsystem 1 kann insofern herstellerunabhängig jedes Untersuchungsgerät als Behandlungsstation eingebunden werden, das im Sinne des DICOM-Standard GPPS-fähig ist.

Anhand des Rückmeldungssignals R überprüft das Terminplanungsmodul 3, ob die einem jeden termingerecht durchgeführten Ablaufschritt 9 zugeordnete Vorgabezeit innerhalb vorgegebener Toleranzgrenzen eingehalten wurde. Solange dies der Fall ist, wird der Terminplan 11 von dem Terminplanungsmodul 3 als eingehalten erkannt.

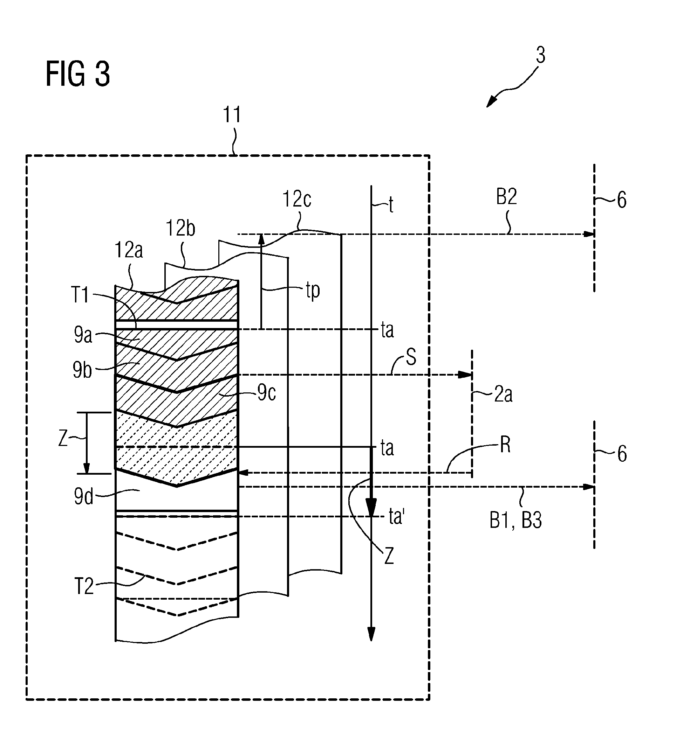

In der klinischen Praxis sind jedoch häufige, und mitunter gravierende Abweichungen der tatsächlichen Behandlungsdauer von der einem standardisierten Ablauf 8a-8c zugewiesenen Vorgabedauer tv unvermeidlich. Ein solcher Fall ist beispielhaft in FIG 3 dargestellt. Hier nimmt die Durchführung des Ablaufschritts 9c des Termins T1 etwa das Dreifache der ihm zugeordneten Vorgabezeit in Anspruch. Zur dynamischen Anpassung der nachfolgenden Termine, insbesondere des anschließenden Termins T2, berechnet das Terminplanungsmodul 3 anhand des Rückmeldungssignals R die tatsächliche Durchführungsdauer des Ablaufschritts 9c des Termins T1. Weiterhin berechnet das Terminplanungsmodul 3 die sich aus der Differenz der tatsächlichen Durchführungsdauer und der Vorgabezeit dieses Ablaufschritts 9c ergebende Zeitverschiebung Z, um welche der Terminplan 11 im dargestellten Beispiel in Verzug geraten ist. Um den Betrag dieser Zeitverschiebung Z verschiebt das Terminplanungsmodul 3 nun den nachfolgenden Ablaufschritt 9d des Termins T1. Weiterhin werden anstelle der ursprünglichen Anfangszeiten ta für alle nachfolgenden Termine T2 um den Betrag der Zeitverschiebung Z korrigierte Anfangszeiten ta' festgesetzt. Die Terminänderung wird dem betroffenen Patienten durch einen von dem Terminplanungsmodul 3 an die entsprechenden Kommunikationseinheiten 6 übermittelte Benachrichtigung B1 automatisch mitgeteilt.

Um zu vermeiden, dass zu behandelnde Patienten bei jeder Terminänderung erneut persönlich benachrichtigt werden und dass infolgedessen ein Patient mit immer neuen Terminänderungen möglicherweise überfrachtet wird, ist optional vorgesehen, dass der einem termingemäß zu behandelnden Patienten zugeordneten Kommunikationseinheit 6 lediglich einmalig, und zwar um eine vorgegebene Vorlaufzeit tp vor der termingemäß angesetzten Anfangszeit ta, eine Benachrichtigung B2 zugeleitet wird. Zumal in einer Klinik ein Patient vergleichsweise spontan zu einer gegebenen Behandlung abrufbar ist, kann diese Vorlaufzeit tp vergleichsweise kurz gewählt sein, so dass das Risiko, dass sich innerhalb dieser kurzen Vorlaufzeit tp eine weitere Verschiebung des Terminplans 11 ergibt, vergleichsweise gering ist. Die Wahrscheinlichkeit langer Wartezeiten für einen Patienten ist somit minimiert. Andererseits wird zweckmäßigerweise bei jeder Änderung des Terminplans 11 eine entsprechende Benachrichtigung B3 mit dem aktualisierten Terminplan 11 an eine Kommunikationseinheit 6 abgegeben, an der ein Patient Information zu dem aktuellen Terminplan 11 bedarfsweise abrufen kann, z.B. an einen auf der Basis von HTML-Dokumenten oder Bildschirmtext betriebenen Informationsbildschirm.

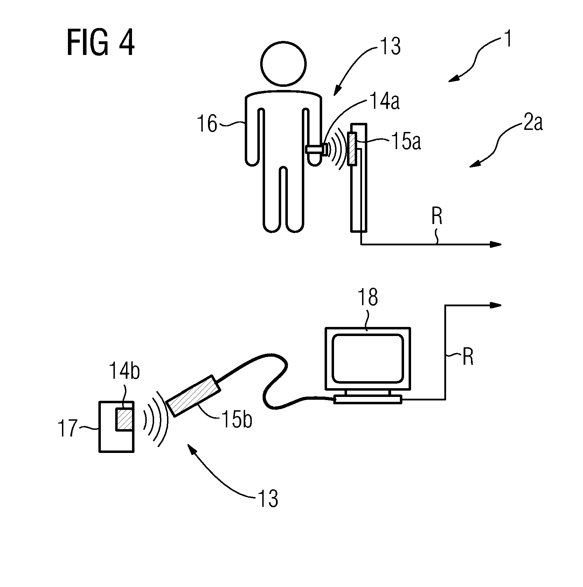

Das Zeitmanagementsystem 1 umfasst bevorzugt ein in FIG 4 schematisch dargestelltes Lokalisationssystem 13 zur Lokalisation von Patienten, Transportgeräten und medizinischen Verbrauchsgütern und zur Generierung entsprechender Rückmeldungssignale R, wenn ein Patient, Transportsystem bzw. Verbrauchsgut lokalisiert ist. Das Lokalisationssystem 13 beruht auf RFID-Technik und umfasst eine Anzahl von RFID-Signalgebern 14a,14b und RFID-Lesegeräten (nachfolgend kurz Lesegeräte) 15a,15b.

Bei einem jedem RFID-Signalgeber 14a,14b handelt es sich um einen (optional aktiven, bevorzugt aber passiven) elektrischen Schwingkreis im Radiowellenfrequenzbereich, der eine vorgegebene Information enthält, die durch elektromagnetische Wechselwirkung von jedem Lesegerät 15a,15b des Lokalisationssystems 13 ausgelesen wird, wenn der RFID-Signalgeber 14a,14b in den Einflussbereich des Lesegeräts 15a,15b gehalten wird.

Die einem RFID-Signalgeber 14a,14b zugeordnete Information ist konfigurierbar, insbesondere änderbar oder löschbar.

Die RFID-Signalgeber 14a,14b sind mobil und jeweils einem Patienten 16, einem Transportgerät oder einer medizinischen Verbrauchsgüterpackung zugeordnet, während die Lesegeräte 15a,15b stationär sind, d.h. einer bestimmten Untersuchungsstation 2a-2c der Klinik zugeordnet sind.

Im dargestellten Beispiel ist ein erster RFID-Signalgeber 14a in einem Armband integriert und an einem Handgelenk des Patienten 16 befestigt. Ein weiterer RFID-Signalgeber 14b ist als Aufkleber realisiert und auf einer Verbrauchsgüterpackung 17 aufgebracht. Weitere RFID-Signalgeber können z.B. in Transportwagen o.dgl. integriert sein.

Ein Lesegerät 15a ist an einem Eingang oder Ausgang eines der Untersuchungsstation 2a zugeordneten Raums, insbesondere Warteraums, eines Umkleideraums oder eines Behandlungszimmers angeordnet. Ein weiteres Lesegerät 15b ist als Handgerät einem Steuerrechner 18 der Untersuchungsstation 2a zugeordnet.

Durch Auslesen der RFID-Signalgeber 14a,14b durch eines der Lesegeräte 15a,15b wird festgestellt, wann der Patient 16 das Lesegerät 15a passiert, indem er z.B. den Warteraum, Umkleideraum bzw. Behandlungsraum, dem dieses Lesegerät 15a zugeordnet ist, betritt oder verlässt. Diese Information wird durch das jeweilige Lesegerät 15a,15b in Form eines entsprechenden Rückmeldungssignals R an das Terminplanungsmodul 3 zurückgegeben und zur dynamischen Anpassung der Terminplanung verwertet.
Ferner wird mittels des Lesegerätes 15b festgestellt, wann und in welchem Umfang medizinische Verbrauchsgüter für die Behandlungsstation 2a eingesetzt werden, etc.

## Patentansprüche

1. Zeitmanagementsystem (1) für medizinische Behandlungen, mit mindestens einer Behandlungsstation (2a-2c) und mit einem Terminplanungsmodul (3), welches dazu ausgebildet ist, eine Anzahl von Terminen (T1,T2) zu speichern, deren jeder einem in Hinblick auf die oder eine bestimmte Behandlungsstation (2a-2c) und eine Vorgabedauer (tv) spezifizierten medizinischen Ablauf (8a-8c) zugeordnet ist,
**dadurch gekennzeichnet,**
- **dass** der oder jeder Ablauf (8a-8c) in eine Anzahl von Ablaufschritten (9,9a-9d) unterteilt ist,
- **dass** die Behandlungsstation (2a-2c) dazu ausgebildet ist, bei vollendeter Durchführung eines jeden Ablaufsschritts (9,9a-9d) ein Rückmeldungssignal (R) an das Terminplanungsmodul (3) auszugeben, und
- **dass** das Terminplanungsmodul (3) dazu ausgebildet ist, nachfolgende Termine (T2) anhand des Rückmeldungssignals (R) dynamisch anzupassen,
- wobei das Terminplanungsmodul (3) zur Terminierung eines Ablaufs (8a-8c) den oder jeden Ablaufschritt (9,9a-9d) des Ablaufs (8a-8c) in Form eines mit dem DICOM-Standard kompatiblen Planned-Procedure-Step formuliert, und
- wobei die oder jede Behandlungsstation (2a-2c) als Rückmeldungssignal (R) einen durchgeführten Ablaufschritt (9,9a-9d) in Form eines mit dem DICOM-Standard kompatiblen Performed-Procedure-Step formuliert.

2. Zeitmanagementsystem (1) nach Anspruch 1,
**dadurch gekennzeichnet, dass** dem Terminplanungsmodul (3) zu der oder jeder Behandlungsstation (2a-2c) mindestens ein in Hinblick auf die zugeordnete Vorgabedauer (tv) spezifizierter Ablauf (8a-8c) vorgegeben ist.

3. Zeitmanagementsystem (1) nach Anspruch 1 oder 2,
**gekennzeichnet durch** ein Lokalisationssystem (13), insbesondere auf Basis eines Strichcode- oder Radiofrequenzidentifikations(RFID)-Systems zur Generierung des Rückmeldungssignals (R).

4. Zeitmanagementsystem (1) nach Anspruch 3,
**dadurch gekennzeichnet, dass** das Lokalisierungssystem (13) mindestens einen RFID-Signalgeber (14a,14b) und mindestens ein RFID-Lesegerät (15a,15b) umfasst, wobei das RFID-Lesegerät (15a, 15b) dazu ausgebildet ist, das Rückmeldungssignal (R) zu erzeugen, wenn der zugehörige RFID-Signalgeber (14a,14b) ausgelesen wird.

5. Zeitmanagementsystem (1) nach Anspruch 4,
**dadurch gekennzeichnet, dass** der oder jeder RFID-Signalgeber (14a,14b) als mobiles Teil ausgebildet ist, während das oder jedes RFID-Lesegerät (15a,15b) stationär ist.

6. Zeitmanagementsystem (1) nach Anspruch 5,
**dadurch gekennzeichnet, dass** mindestens einer der RFID-Signalgeber (14a,14b) einem Patienten (16), einem Transportgerät oder einem medizinischen Verbrauchsgut (17) zuordenbar ist.

7. Zeitmanagementsystem (1) nach einem der Ansprüche 1 bis 6,
**dadurch gekennzeichnet, dass** das Terminplanungsmodul (3) dazu ausgebildet ist, im Zuge der Terminierung eines Ablaufs (8a-8c) die dem Termin (T1,T2) zugeordnete Anfangszeit (ta) automatisch auszuwählen.

8. Zeitmanagementsystem (1) nach einem der Ansprüche 1 bis 7,
**dadurch gekennzeichnet, dass** das Terminplanungsmodul (3) dazu ausgebildet ist, bei einer dynamischen Anpassung eines Termins (T1,T2) einen diesem zugeordneten Patienten automatisch zu benachrichtigen.

9. Zeitmanagementsystem (1) nach einem der Ansprüche 1 bis 8,
**dadurch gekennzeichnet, dass** das Terminplanungsmodul (3) dazu ausgebildet ist, einen einem Termin (T1,T2) zugeordneten Patienten eine vorgegebene Vorlaufzeitspanne (tp) vor einer dem Termin (T1,T2) zugeordneten Anfangszeit (ta,ta') automatisch zu benachrichtigen.
